# EUROPEAN PATENT SPECIFICATION

(11) **EP 4 213 918 B1**
(45) Date of publication and mention of the grant of the patent: **09.04.2025**
(21) Application number: 21773663.6
(22) Date of filing: 15.09.2021
(51) Int. Cl.: A61M 5/28, B65D 81/32, A61M 5/31

(54) **DEFORMABLE PRE-PACKAGED DEVICE FOR INJECTING A LIQUID**
VERFORMBARE VORVERPACKTE VORRICHTUNG ZUM EINSPRITZEN EINER FLÜSSIGKEIT
DISPOSITIF PRÉ-EMBALLÉ DÉFORMABLE POUR L'INJECTION D'UN LIQUIDE

(30) Priority: 21.09.2020 IT 202000022168
(43) Date of publication of application: 26.07.2023
(73) Proprietor: Orofino Pharmaceuticals Group SRL, 00193 Roma (IT)
(72) Inventor: OROFINO, Ernesto, 00193 Roma (IT)
(74) Representative: Perronace, Andrea
(86) International application number: PCT/IB2021/058406
(87) International publication number: WO 2022/058906

(56) References cited:
- WO-A1-2019/246435
- WO-A1-2020/070577
- US-A- 4 410 323

## Description

The present invention relates to a deformable pre-packaged device for injecting a liquid.

### Background art

Currently, sterile powdered raw materials are mainly packaged by active ingredient manufacturers in aluminum containers, or in plastic bags, generally based on polyethylene.

In order to market them, the producers have shown that each of the raw materials, in addition to maintaining sterility, remains stable inside the .containers or bags used for a certain period of time; in other words, such powders do not degrade, for a predefined number of years, if stored in such containers or bags.

The sterile raw materials are sold to the producers of the finished pharmaceutical product, who see to the fractionation using known technologies, placing the finished sterile powder product contained in a bottle on the market. The bottle is accompanied by solvent.

The use of valuable materials for the vial (glass), of compatible materials for the sterile powder, the use of disposable syringes, make this system globally very expensive.

It is known in the field to use syringes provided with a compartment for receiving a deformable cartridge, made of flexible material, containing pharmaceutical substances kept separate in respective containment chambers until the moment of use by a special yielding partition. An example of such known devices is described in patent document US2001/0047162. A further example of such known devices is described in patent document WO2017137854 A1.

However, it has been observed that in the deformable cartridges of the prior art it is particularly difficult to maintain a high degree of isolation between the various containment chambers. This is particularly problematic if the containment chambers of the deformable cartridge are filled with mutually different substances, for example a solid substance and a liquid substance or for example two substances which, if accidentally mixed together earlier than expected, cause a stability loss of the active ingredients thereof.

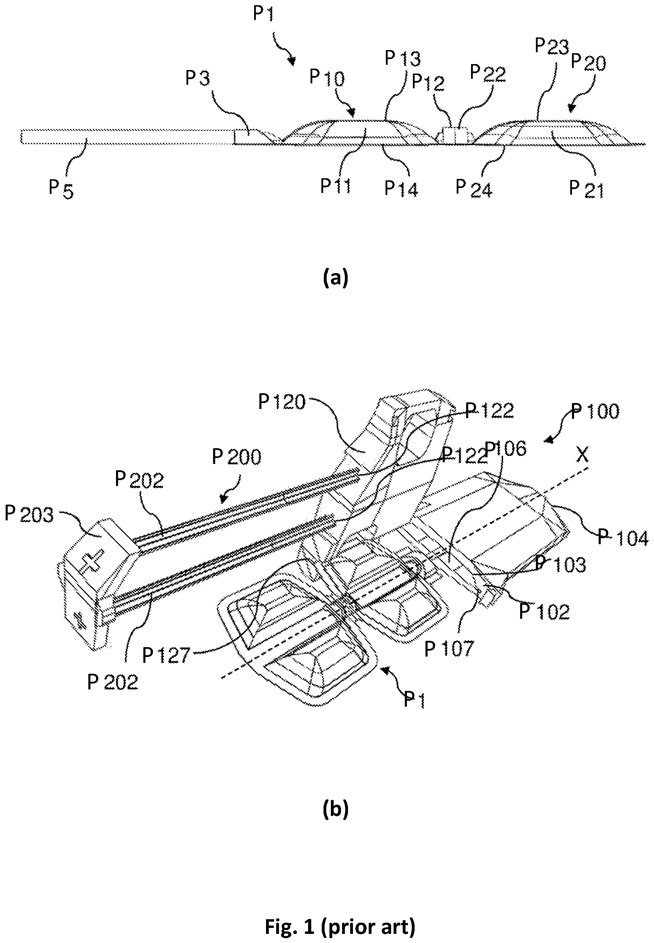

Patent document WO 2020/070576 to the Applicant, shown in Fig. 1, provides a solution by means of the deformable cartridge P1 which has a cartridge body P10, P20 comprising (Fig. 1(a)):
- at least a first containment chamber P11 of a first component of the injectable solution, in which the first containment chamber P11 comprises at least a first wall deformable by compression;
- at least a second containment chamber P21 of a second component of the injectable solution, in which the second containment chamber P21 comprises at least a second wall deformable by compression so as to allow the transfer of the second component from the second containment chamber P21 to the first containment chamber P11, thus achieving the mixing of the first component and the second component; and
- a needle P5 connected to the first containment chamber P11.

As shown in Fig. 1(b), such a device P1 is to be used with a specific tool P200 to deform the chambers so as to mix the components and the simultaneous injection. This involves the production of a specific element and therefore a considerable investment in molds and materials, as well as a certain difficulty of use by the operator who performs the injection.

In particular, with regard to the difficulty of use, it is not only a question of the use of the specific tool for crushing the chambers, but also the fact that the operator or nurse will have a reduced sensitivity in pointing and inserting the needle with respect to what is obtainable by means of traditional syringes in which the mixture to be injected is sucked and the syringe held with only one hand.

Document WO 2019/246435 concerns a bellows device for ejecting a liquid substance, comprising two chambers, which are not separately crushable and therefore which are not adapted to separately store and reconstitute a substance starting from two components.

### Purpose and object of the invention

It is the object of the present invention to provide a deformable pre-packaged device for injecting a liquid which at least partially solves the problems and overcomes the disadvantages of the prior art.

The present invention relates to a device and a corresponding kit according to the appended claims.

### Detailed description of embodiments of the invention

### List of drawings

The invention will now be described by way of non-limiting example, with particular reference to the figures of the accompanying drawings, in which:
- figure 1 shows a reconstitution and injection device according to the prior art;
- figure 2 shows a vertical sectional view of an embodiment of the invention;
- figure 3 shows the device in Fig. 2 in successive steps of use (a)-(d);
- figure 4 shows the same device in the same steps as in Fig. 3, but not in section.

It is worth noting that hereinafter elements of different embodiments may be combined together to provide further embodiments without restrictions respecting the technical concept of the invention, as those skilled in the art will effortlessly understand from the description.

The present description also refers to the prior art for the implementation thereof, with regard to the detail features which are not described, such as elements of minor importance usually used in the prior art in solutions of the same type.

When an element is introduced, it is always understood that there may be "at least one" or "one or more".

When a list of elements or features is given in this description, it is understood that the finding according to the invention "comprises" or alternatively "consists of" such elements.

When "upper" and "lower" are mentioned, reference is made to the next location along an axis, which will be specified. In particular, "lower" means a location farther away from the outlet of a fluid from a containment element and "upper" means a location closer to the outlet of a fluid from a containment element.

### Embodiments

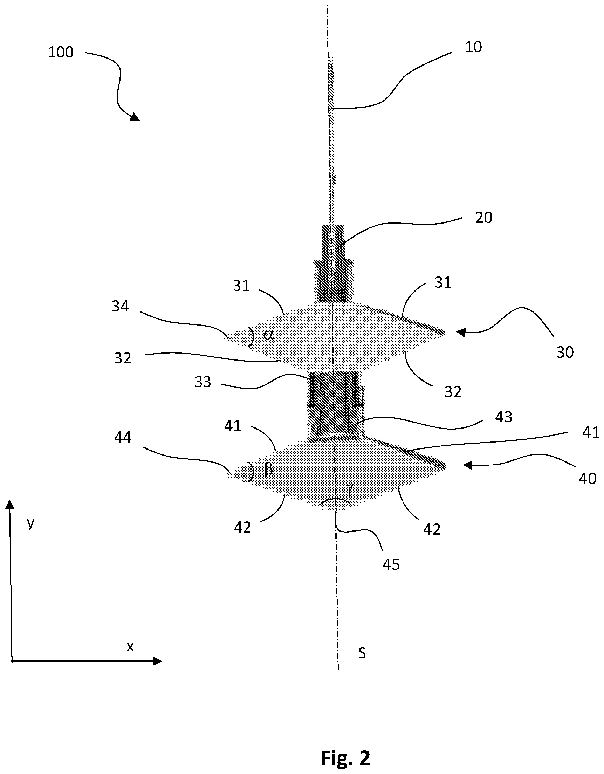

With reference to Figure 2, in an embodiment, the injection device 100 of the invention comprises:
- a first containment chamber 40 for a first component of a liquid mixture to be injected;
- a second containment chamber 30 for a second component (for example in powder form, in the drawings rendered with the dotted line) of the liquid mixture to be injected;
which are fluidly connected by (rigid or semi-rigid) mechanical and fluidic connection means 33, 43.

In greater detail, such (first) mechanical and fluidic connection means can consist of a rigid or semi-rigid tubular outlet element (or "neck") 43 connected to (or formed in one piece with) the first containment chamber 40, and a tubular inlet element 33 connected to (or formed in one piece with) the second containment chamber 30.

A one-way valve can optionally be added to one of the elements 33 or 43 so as to allow the passage of the liquid from the chamber 40 and the retention thereof in the chamber 30.

The tubular outlet element 43 and the tubular inlet element 33 are provided with appropriate mutual mechanical coupling means (not shown), preferably watertight (without fluid leaks). The tubular elements 33 and 43 can have any section, as long as the fluidic connection can be made in a watertight manner. When the first containment chamber and the second containment chamber are provided, separated, in an injection kit (optionally also comprising the needle, it being possible to provide other means of administration or discharge of the mixture such as tubes), such tubular elements can comprise breakable or openable or removable watertight closing elements, such as peelable partitions.

The second containment chamber also comprises a tubular outlet element 20 (also referred to as "second mechanical and fluidic connection means") configured so that an injection needle 10 can be fixed thereto (in a watertight manner, i.e., without fluidic communication with the outside). When the second containment chamber is provided in the above kit, the tubular element (or the second mechanical and fluidic connection means) is provided with a removable watertight closing element, such as a peelable partition.

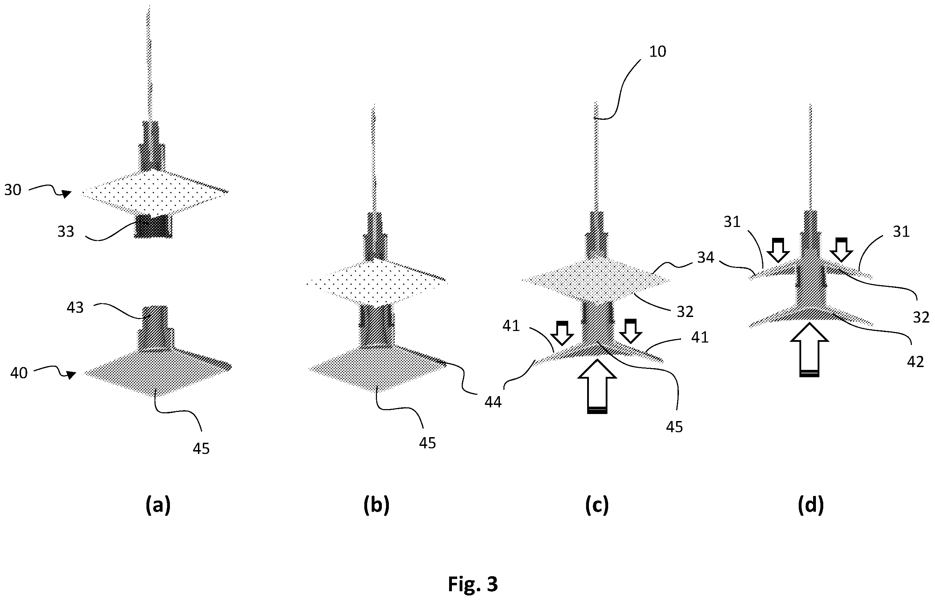

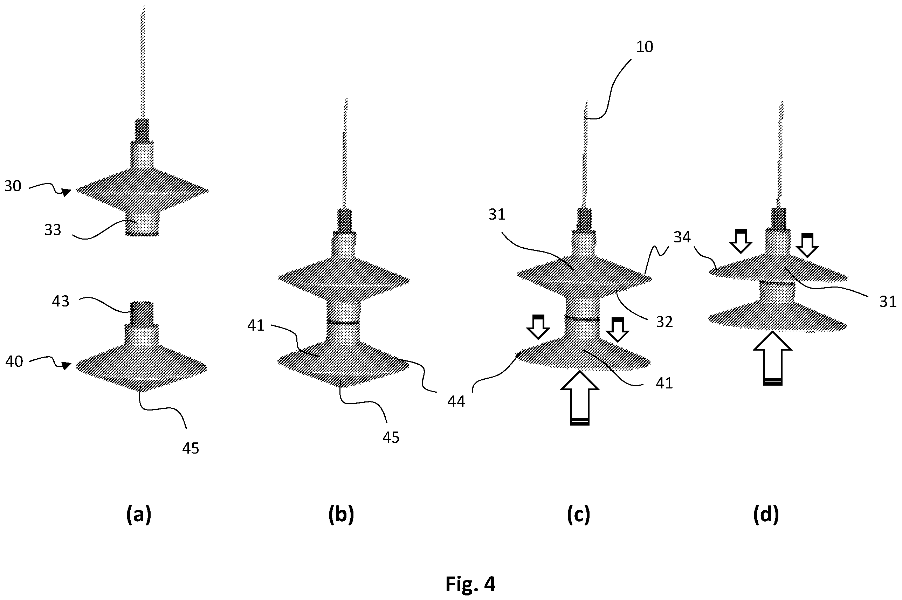

As mentioned, the first 40 and the second 30 containment chambers are configured so that they can be stored separately, therefore with the tubular elements 33 and 43 detached, and joined when the injection is to be made (see Figures 3 (a) and 4 (a)). The combination of the elements 33 and 43 can occur in any known manner, for example by means of threading and/or breakable membranes.

One or both of the tubular elements 33 and 43 can comprise a filter and/or a valve, for example a valve adapted to allow a one-way flow of said first component of the liquid mixture.

The first mechanical and fluidic connection means 43,33 comprise an outlet neck of said first containment chamber 40 and an inlet neck of said second containment chamber 30 connected to form a single fluidic connection duct; and

Said single fluidic connection duct is such as to keep said first lower surface 42 and said second lower surface 32 at a distance configured to be larger than the section of a finger. The section of a finger can be standardized to a size such as to comprise all human fingers or most of them.

The first containment chamber 40 preferably has, in vertical section (plane x-y of the sheet), a diamond shape or cone section or truncated cone section. For example, in the first containment chamber 40, the angle β between the upper 41 and lower 42 sides (on one side as well as on the other side of the diamond in the direction x) has a predetermined (non-zero) value before the use of the device. The same applies to the lower tip angle (vertex of the cone 45) γ between two sides 42 (on one side as well as on the other side of the diamond in the direction x), with the pre -determined (non-zero) value thereof before the use of the syringe 100. The upper tip angle (in the direction y) may not exist in this case, because the tubular outlet element 43 is fixed in the position of such vertices. In reality, the lower tip 45 may not exist as well, as a flat or other shaped part may be formed, on which the thumb will push, as explained below.

The second containment chamber 30 also has a diamond shape, and in this case the angle α between the upper 31 and lower 32 sides (on one side as well as on the other side of the diamond in the direction x) has a predetermined value thereof before the use of the device. The lower or upper tip angle may not exist in this case, because the tubular inlet 33 and outlet 20 elements are fixed in the position of such vertices.

In the above, it is understood that the shape of the containment chamber has, in the volume device, a circular extension along an axis of symmetry S (in the direction y) of the syringe 100, i.e., the section is rotated by 180° to obtain the volume of the containment chambers (or each corresponding upper and lower side is rotated by 360°), to obtain an overall shape which corresponds to the combination of two cones with opposite vertices (or truncated cones or the like) along a common base 44 or 34.

Furthermore, the surfaces obtained from the above rotation do not necessarily have a perfectly conical shape, but can be more or less rounded, because this does not much change the functionality thereof.

In fact, the double cone shape (diamond or the like in section) has a very specific function, namely that of allowing the containment chambers to be crushed with the fingers according to determined steps illustrated without limitation in Figures 3 and **4****.**

Specifically, according to an embodiment of the present invention, after the mechanical and fluidic joining of the first and second containment chambers 40 and 30 (Figures 3(b) and 4(b)), the operator (or nurse) who must perform the parenteral (or other) administration places the index and middle fingers (or two fingers other than the thumb) of a hand above the diamond 40 (on the sides 41) while pressing the lower surface of the diamond with the thumb (sides 42, see also the arrows in the figure) until the latter are crushed against the former, making the angle γ an angle approximately equal to 180° - γ, and thus allowing the liquid component of the first containment chamber to enter the second containment chamber. Since the material of the diamond 40 is inelastically deformable (deformable by compression), such crushing will remain even after loosening the thrust between the index and middle fingers on one side and thumb on the other. This will allow (Fig. 3/4(d)) to reposition the index and middle fingers (or the other two fingers other than the thumb) again on the upper surface of the containment chamber 30, i.e., on the sides 31 in section, while the thumb will be able to continue pushing in the same position as before. Similarly to the previous step, the lower surface of the containment chamber 30 (walls 32 in section) will be pressed (see the arrows in the figure) against the corresponding upper surface (walls 31 in section). Here, as well, the material used will allow inelastic deformation, so as to ensure the safety of the injection.

According to an aspect of the invention, said first lower surface 42 and said second lower surface 32 in the device 100 are made of an inelastically deformable material. In particular, the inelastically deformable material is such as to allow a total crushing of said first lower surface 42 and said second lower surface 32 against said first upper surface 41 and second upper surface 31, respectively in the various steps of reconstituting and ejecting the mixture. The deformability is separately ensured for each chamber 40 and 30.

The needle 10 of the syringe 100 can be conveniently introduced into the body to receive the administration after step (c) in the figures, or after the two components in the two containment chambers have been reconstituted in the first containment chamber. Obviously, the corresponding initial filling of the two containment chambers (and therefore the sizing thereof) will be such as to perform the whole operation.

According to an aspect of the invention, a method for reconstituting a reconstitutable liquid mixture and ejecting it from an injection needle is provided, comprising the following successive steps:
- providing the device according to the invention;
- positioning two fingers on said first upper surface 41 and the thumb against said first lower surface 42;
- crushing said lower surface 42 against said first upper surface 41 bringing the thumb closer to said two fingers;
- positioning two fingers on said second upper surface 31 and the thumb against said first lower surface 42; and
- crushing said second upper surface 31 against said second lower surface 32 bringing the thumb closer to said two fingers.

### Advantages of the invention

By virtue of the solution of the invention, it is possible to store two components of a mixture to be injected, in two separate connectable devices, and to reconstitute the syringe and the mixture at the time of injection.

This is done, by virtue of the invention, without having to use any device other than the syringe itself, since the means for crushing the containment chambers of the two components are integrated in the syringe itself.

Such a solution allows to save the costs of the molds and the production of the separate crushing and reconstitution means, as well as simplify the reconstitution and injection operations. In particular, with the device according to the invention the operator or nurse will have a needle pointing and insertion sensitivity which is similar to or better than that obtainable by means of traditional syringes into which the mixture to be injected is sucked.

The preferred embodiments and possible variants of the present invention have been outlined above, but it is to be understood that those skilled in the art may make modifications and changes without thereby departing from the scope of protection thereof, as defined in the appended claims.

## Claims

1. A device (100) for injecting a reconstitutable liquid mixture, comprising:
- a first containment chamber (40) for a first component of the liquid mixture;
- at least a second containment chamber (30) for a second component of the liquid mixture,
- first mechanical and fluidic connection means (43,33) between said first (40) and said second (30) containment chambers;
- second mechanical and fluidic connection means (20) between said second containment chamber (30) and an injection needle (10);
the device (100) being **characterized in that**:
- said first containment chamber (40) comprises, along a mixture injection direction (y), a first upper surface (41) and a first lower surface (42), in the shape of a cone or truncated cone, substantially having the same axis of symmetry (S) and a common base circumference (44);
- said first upper surface (41) and said first lower surface (42) have extensions in opposite directions along said axis of symmetry (S) and form a non-zero angle β therebetween at the common base (44);
- said second containment chamber (30) comprises, along said mixture injection direction (y), a second upper surface (31) and a second lower surface (32), in the shape of a cone or truncated cone, both substantially having said axis of symmetry (S) and a common base circumference (34),
- said second upper surface (31) and said second lower surface (32) have extensions in opposite directions along said axis of symmetry (S) and form a non-zero angle α therebetween at the common base (34);
- said first lower surface (42) and said second lower surface (32) are separately deformable by compression;
and **in that**:
- said first mechanical and fluidic connection means (43,33) comprise an outlet neck of said first containment chamber (40) and an inlet neck of said second containment chamber (30) connected to form a single fluidic connection duct; and
- said single fluidic connection duct is such as to keep said first lower surface (42) and said second lower surface (32) at a distance configured to be larger than the section of a finger;
wherein:
- said first lower surface (42) and said second lower surface (32) are made of an inelastically deformable material;
- said inelastically deformable material is such as to allow a total crushing of said first lower surface (42) and said second lower surface (32) against said first upper surface (41) and second upper surface (31), respectively.

2. A device (100) according to claim 1, wherein said first mechanical and fluidic connection means (43,33) comprise a filter and/or a valve.

3. A device (100) according to claim 2, wherein said first mechanical and fluidic connection means (43,33) comprise a valve adapted to allow a one-way flow of said first component of the liquid mixture.

4. A device (100) according to one or more of claims 1 to 3, wherein said first upper surface (41) and said first lower surface (42) are formed in one piece.

5. A device (100) according to one or more of claims 1 to 4, wherein said second upper surface (31) and said second lower surface (32) are formed in one piece.

6. An injection kit for injecting a reconstitutable liquid mixture, comprising:
- a first containment chamber (40) and a second separate containment chamber (30), as individually defined in one of claims 1 to 5;
- first mechanical and fluidic connection means (43,33) between the first containment chamber (40) and the second containment chamber (30) according to claim 1, wherein said outlet neck of said first containment chamber (40) and said inlet neck of said second containment chamber (30) are configured to connect and form a single fluidic connection duct;
- second mechanical and fluidic connection means (20) between said second containment chamber (30) and an injection needle (10);
- said injection needle (10) which is connectable to said second containment chamber (30) by said second mechanical and fluidic connection means (20).

7. An injection kit according to claim 6, wherein said outlet neck of said first containment chamber (40) and said inlet neck of said second containment chamber (30) each comprise a removable or breakable or openable watertight closing element.

8. An injection kit according to claim 7, wherein the removable watertight closing element is a peelable partition.

9. An injection kit according to one or more of claims 6 to 8, wherein said second mechanical and fluidic connection means (20) comprise a removable watertight closing element.

10. A method for reconstituting a reconstitutable liquid mixture comprising the following successive steps:
- providing the device according to one or more of claims 1 to 5;
- positioning two fingers on said first upper surface (41) and the thumb against said first lower surface (42);
- crushing said first lower surface (42) against said first upper surface (41) bringing the thumb closer to said two fingers.

## Patentansprüche

1. Vorrichtung (100) zum Einspritzen einer rekonstituierbaren flüssigen Mischung, umfassend:
- eine erste Rückhaltekammer (40) für eine erste Komponente der flüssigen Mischung;
- mindestens eine zweite Rückhaltekammer (30) für eine zweite Komponente der flüssigen Mischung,
- erste mechanische und fluidische Verbindungsmittel (43, 33) zwischen der ersten (40) und der zweiten (30) Rückhaltekammer;
- zweite mechanisches und fluidische Verbindungsmittel (20) zwischen der zweiten Rückhaltekammer (30) und einer Einspritznadel (10);
wobei die Vorrichtung (100) **dadurch gekennzeichnet ist, dass**:
- die erste Rückhaltekammer (40) entlang einer Einspritzrichtung (y) einer Mischung eine erste obere Fläche (41) und eine erste untere Fläche (42) in der Form eines Kegels oder Kegelstumpfes umfasst, die im Wesentlichen dieselbe Symmetrieachse (S) und einen gemeinsamen Grundumfang (44) aufweisen;
- die erste obere Fläche (41) und die erste untere Fläche (42) Verlängerungen in entgegengesetzten Richtungen entlang der Symmetrieachse (S) aufweisen und einen von Null verschiedenen Winkel β zwischen sich an der gemeinsamen Basis (44) bilden;
- die zweite Rückhaltekammer (30) entlang der Einspritzrichtung (y) der Mischung eine zweite obere Fläche (31) und eine zweite untere Fläche (32) in der Form eines Kegels oder Kegelstumpfes umfasst, wobei beide im Wesentlichen die Symmetrieachse (S) und einen gemeinsamen Basisumfang (34) aufweisen,
- die zweite obere Fläche (31) und die zweite untere Fläche (32) Verlängerungen in entgegengesetzten Richtungen entlang der Symmetrieachse (S) aufweisen und zwischen sich an der gemeinsamen Basis (34) einen von Null verschiedenen Winkel α bilden;
- die erste untere Fläche (42) und die zweite untere Fläche (32) durch Kompression separat verformbar sind;
und dadurch, dass:
- die ersten mechanischen und fluidischen Verbindungsmittel (43, 33) einen Auslasshals der ersten Rückhaltekammer (40) und einen Einlasshals der zweiten Rückhaltekammer (30) umfassen, die verbunden sind, um ein einzelnes fluidisches Verbindungsrohr zu bilden; und
- das einzelne fluidische Verbindungsrohr so beschaffen ist, dass es die erste untere Fläche (42) und die zweite untere Fläche (32) in einer Distanz hält, die so konfiguriert ist, dass sie größer als der Querschnitt eines Fingers ist;
wobei:
- die erste untere Fläche (42) und die zweite untere Fläche (32) aus einem unelastisch verformbaren Material bestehen;
- das unelastisch verformbare Material so beschaffen ist, dass es ein vollständiges Zusammendrücken der ersten unteren Fläche (42) und der zweiten unteren Fläche (32) gegen die erste obere Fläche (41) bzw. die zweite obere Fläche (31) ermöglicht.

2. Vorrichtung (100) nach Anspruch 1, wobei die ersten mechanischen und fluidischen Verbindungsmittel (43, 33) einen Filter und/oder ein Ventil umfassen.

3. Vorrichtung (100) nach Anspruch 2, wobei die ersten mechanischen und fluidischen Verbindungsmittel (43, 33) ein Ventil umfassen, das geeignet ist, einen Einwegfluss der ersten Komponente der flüssigen Mischung zu ermöglichen.

4. Vorrichtung (100) nach einem oder mehreren der Ansprüche 1 bis 3, wobei die erste obere Fläche (41) und die erste untere Fläche (42) einstückig ausgebildet sind.

5. Vorrichtung (100) nach einem oder mehreren der Ansprüche 1 bis 4, wobei die zweite obere Fläche (31) und die zweite untere Fläche (32) einstückig ausgebildet sind.

6. Einspritzset zum Einspritzen einer rekonstituierbaren flüssigen Mischung, umfassend:
- eine erste Rückhaltekammer (40) und eine zweite separate Rückhaltekammer (30), wie in einem der Ansprüche 1 bis 5 einzeln definiert;
- erste mechanische und fluidische Verbindungsmittel (43, 33) zwischen der ersten Rückhaltekammer (40) und der zweiten Rückhaltekammer (30) nach Anspruch 1, wobei der Auslasshals der ersten Rückhaltekammer (40) und der Einlasshals der zweiten Rückhaltekammer (30) so ausgelegt sind, dass sie verbunden werden und ein einzelnes fluidisches Verbindungsrohr bilden;
- zweite mechanische und fluidische Verbindungsmittel (20) zwischen der zweiten Rückhaltekammer (30) und einer Einspritznadel (10);
- die Einspritznadel (10), die mit der zweiten Rückhaltekammer (30) durch die zweiten mechanischen und fluidischen Verbindungsmittel (20) verbunden werden kann.

7. Einspritzset nach Anspruch 6, wobei der Auslasshals der ersten Rückhaltekammer (40) und der Einlasshals der zweiten Rückhaltekammer (30) jeweils ein abnehmbares, zerbrechliches oder zu öffnendes wasserdichtes Verschlusselement umfassen.

8. Einspritzset nach Anspruch 7, wobei das abnehmbare wasserdichte Verschlusselement eine abziehbare Trennwand ist.

9. Einspritzset nach einem oder mehreren der Ansprüche 6 bis 8, wobei die zweiten mechanischen und fluidischen Verbindungsmittel (20) ein abnehmbares wasserdichtes Verschlusselement umfassen.

10. Verfahren zum Wiederherstellen einer rekonstituierbaren flüssigen Mischung, umfassend die folgenden aufeinanderfolgenden Schritte:
- Bereitstellen der Vorrichtung nach einem oder mehreren der Ansprüche 1 bis 5;
- Positionieren von zwei Fingern auf der ersten oberen Fläche (41) und des Daumens gegen die erste untere Fläche (42);
- Drücken der ersten unteren Fläche (42) gegen die erste obere Fläche (41), wobei der Daumen näher an die zwei Finger gebracht wird.

## Revendications

1. Dispositif (100) d'injection d'un mélange liquide reconstituable, comprenant :
- une première chambre de confinement (40) pour un premier composant du mélange liquide ;
- au moins une seconde chambre de confinement (30) pour un second composant du mélange liquide,
- des premiers moyens de raccordement mécanique et fluidique (43, 33) entre lesdites première (40) et seconde (30) chambres de confinement ;
- des seconds moyens de raccordement mécanique et fluidique (20) entre ladite seconde chambre de confinement (30) et une aiguille d'injection (10) ;
le dispositif (100) étant **caractérisé en ce que** :
- ladite première chambre de confinement (40) comprend, selon un sens d'injection de mélange (y), une première surface supérieure (41) et une première surface inférieure (42), en forme de cône ou de cône tronqué, ayant sensiblement le même axe de symétrie (S) et une circonférence de base commune (44) ;
- ladite première surface supérieure (41) et ladite première surface inférieure (42) ont des extensions dans des sens opposés le long dudit axe de symétrie (S) et forment un angle β non nul entre elles au niveau de la base commune (44) ;
- ladite seconde chambre de confinement (30) comprend, selon ledit sens d'injection de mélange (y), une seconde surface supérieure (31) et une seconde surface inférieure (32), en forme de cône ou de cône tronqué, toutes deux présentant sensiblement ledit axe de symétrie (S) et une circonférence de base commune (34),
- ladite seconde surface supérieure (31) et ladite seconde surface inférieure (32) ont des extensions dans des sens opposés le long dudit axe de symétrie (S) et forment un angle α non nul entre elles au niveau de la base commune (34) ;
- ladite première surface inférieure (42) et ladite seconde surface inférieure (32) sont déformables séparément par compression ;
et **en ce que** :
- lesdits premiers moyens de raccordement mécanique et fluidique (43, 33) comprennent un col de sortie de ladite première chambre de confinement (40) et un col d'entrée de ladite seconde chambre de confinement (30), reliés pour former un seul conduit de raccordement fluidique ; et
- ledit conduit de raccordement fluidique unique est tel qu'il maintient ladite première surface inférieure (42) et ladite seconde surface inférieure (32) à une distance conçue pour être plus grande que la section d'un doigt ;
dans lequel :
- ladite première surface inférieure (42) et ladite seconde surface inférieure (32) sont constituées d'un matériau inélastiquement déformable ;
- ledit matériau inélastiquement déformable est tel qu'il permet un écrasement total de ladite première surface inférieure (42) et de ladite seconde surface inférieure (32) respectivement contre ladite première surface supérieure (41) et contre ladite seconde surface supérieure (31).

2. Dispositif (100) selon la revendication 1, dans lequel lesdits premiers moyens de raccordement mécanique et fluidique (43, 33) comprennent un filtre et/ou une soupape.

3. Dispositif (100) selon la revendication 2, dans lequel lesdits premiers moyens de raccordement mécanique et fluidique (43, 33) comprennent une soupape adaptée pour permettre un écoulement unidirectionnel dudit premier composant du mélange liquide.

4. Dispositif (100) selon une ou plusieurs des revendications 1 à 3, dans lequel ladite première surface supérieure (41) et ladite première surface inférieure (42) sont formées d'une seule pièce.

5. Dispositif (100) selon une ou plusieurs des revendications 1 à 4, dans lequel ladite seconde surface supérieure (31) et ladite seconde surface inférieure (32) sont formées d'une seule pièce.

6. Kit d'injection permettant d'injecter un mélange liquide reconstituable, comprenant :
- une première chambre de confinement (40) et une seconde chambre de confinement (30) séparée, telles que définies individuellement dans l'une des revendications 1 à 5 ;
- des premiers moyens de raccordement mécanique et fluidique (43, 33) entre la première chambre de confinement (40) et la seconde chambre de confinement (30) selon la revendication 1, dans lequel ledit col de sortie de ladite première chambre de confinement (40) et ledit col d'entrée de ladite seconde chambre de confinement (30) sont conçus pour être raccordés en formant un seul conduit de raccordement fluidique ;
- des seconds moyens de raccordement mécanique et fluidique (20) entre ladite seconde chambre de confinement (30) et une aiguille d'injection (10) ;
- ladite aiguille d'injection (10) qui est raccordable à ladite seconde chambre de confinement (30) par lesdits seconds moyens de raccordement mécanique et fluidique (20).

7. Kit d'injection selon la revendication 6, dans lequel ledit col de sortie de ladite première chambre de confinement (40) et ledit col d'entrée de ladite seconde chambre de confinement (30) comprennent chacun un élément de fermeture étanche amovible, cassable ou ouvrable.

8. Kit d'injection selon la revendication 7, dans lequel l'élément de fermeture étanche amovible est une cloison pelable.

9. Kit d'injection selon une ou plusieurs des revendications 6 à 8, dans lequel lesdits seconds moyens de raccordement mécanique et fluidique (20) comprennent un élément de fermeture étanche amovible.

10. Procédé de reconstitution d'un mélange liquide reconstituable comprenant les étapes successives suivantes :
- la fourniture du dispositif selon une ou plusieurs des revendications 1 à 5 ;
- le positionnement de deux doigts sur ladite première surface supérieure (41) et du pouce contre ladite première surface inférieure (42) ;
- l'écrasement de ladite première surface inférieure (42) contre ladite première surface supérieure (41), rapprochant ainsi le pouce desdits deux doigts.
